# EUROPEAN PATENT APPLICATION

(11) **EP 3 181 562 A1**
(43) Date of publication of application: **21.06.2017**
(21) Application number: 15831693.5
(22) Date of filing: 10.08.2015
(51) Int. Cl.: C07D 405/14, A61P 35/02

(54) **PHENYLAMINOPYRIMIDINE-DERIVED COMPOUNDS, METHOD FOR PRODUCING THE SAME, USE OF SAID COMPOUNDS FOR THE TREATMENT OF CANCER, AND TREATMENT METHODS**

(30) Priority: 11.08.2014 BR 102014019808
(71) Applicant: Fundacao Oswaldo Cruz - Fiocruz, Manguinhos, CEP-21045-900 Rio de Janeiro (BR)
(72) Inventor: BOECHAT, Nubia, CEP:21358-000 Rio de Janeiro, RJ (BR); BASTOS, Mónica Macedo, CEP: 21330-550 Vila Valqueire, RJ (BR); MAIA, Raquel Ciuvalschi, CEP: 20521-050 Rio de Janeiro, RJ (BR)
(74) Representative: Grünecker Patent- und Rechtsanwälte PartG mbB
(86) International application number: PCT/BR2015/000125
(87) International publication number: WO 2016/023090

(57) **Abstract**

The present invention relates to novel phenylaminopyrimidine(FAP)-derived compounds of general formulas I and II: 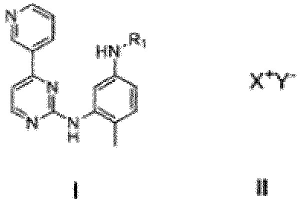 Where, in formula I, R₁ is: 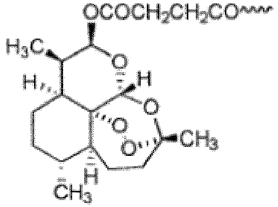 Where, in formula II, X⁺ is selected among one of the compounds below: 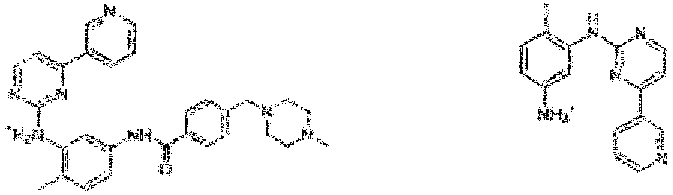 Where, in formula II, Y is

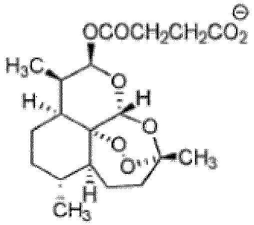

The compounds of the present invention are powerful and nonspecific tyrosine kinase inhibitors, and the use of these compounds in the treatment.

## Description

### FIELD OF THE INVENTION

The present invention relates to novel phenylaminopyrimidine(FAP)-derived compounds of general formulas I and II: 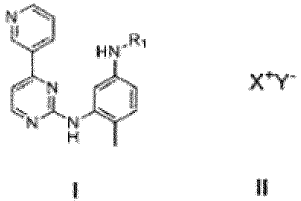 Where, in formula I, R₁ is: 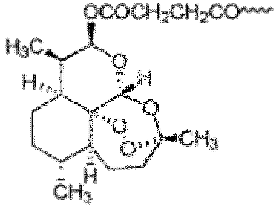 Where, in formula II, X⁺ is selected among one of the compounds below: 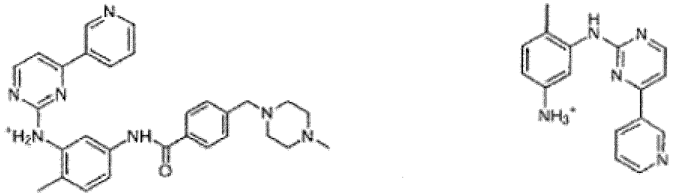 Where, in formula II, Y- is

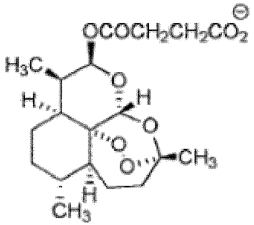

The compounds of formulas I and II of the present invention are powerful and nonspecific inhibitors of tyrosine kinase, being based on hybrid structures between artesunate with the phenylaminopyrimidine (FAP) backbone and imatinib, which can be amides or salts derived therefrom, respectively. These are innovative analogues of imatinib, with high antineoplastic activity and low toxicity, which can be transformed into new options for the treatment of patients with chronic myelogenous leukemia (CML).

### BACKGROUND OF THE INVENTION

Cancer is a generic term to denote a group of diseases having in common the disordered growth of abnormal cells. A characteristic of cancer is the rapid multiplication of these cells, which extend beyond their usual limits and can invade adjacent parts of the body or spread to other organs, wherein this process is known as metastasis.

Cancer is one of the leading causes of death in the world, being responsible for 13% of total deaths. Approximately 30% of cancer deaths are due to four main risk factors: high body mass index, reduction of fruit and vegetable intake, lack of physical activity, high consumption of tobacco and alcohol.

Cancer is a neoplasm that is included in a group of diseases called non-transmissible chronic diseases (NTCD). The types of cancer with the highest occurrence of deaths in the world population are: lung cancer, with 1.37 million/year; stomach cancer, with 736 thousand/year; liver, with 695 thousand/year; colorectal cancer, with 608 thousand/year; breast cancer, with 458 thousand/year, and cervical cancer, with 275 thousand/year (WHO, 2013).

Leukemia is a type of cancer with medium incidence in adults. The term leukemia originates from Greek and means white blood. It is a neoplasm whose characteristic is the accumulation of abnormal young cells in the bone marrow, which replace the normal blood cells.

Leukemia is classified according to the type of cell involved and the rate of progression of the disease (Fauci *et al.,* 2008). If the affected cells are lymphoid, lymphatic or forming lymphoid tissue, it is called lymphoid leukemia. When the target are the myelogenous cells (cells that later become leukocytes), it is called myelogenous leukemia. The progression of the disease classifies it as acute or chronic leukemia. In the chronic phase, the cells can still perform the functions of the white globules, replicating more slowly, and being able to function for a longer period. Some forms of chronic leukemia do not present initial symptoms, and diagnosis may not occur during the first few years. The acute phase begins after some white blood cells lose or damage their sequence of deoxyribonucleic acids (DNA), remaining immature. These are known as blastic cells, which still maintain their ability to multiply. These cells do not die as normal ones, and when they accumulate, they interfere with the functioning of vital organs. In addition, they do not perform their functions, as they multiply faster than normal, worsening the disease in a short time. Acute leukemias require rapid and aggressive treatment (Rodriguez *et al.,* 2006).

Leukemias can be classified into four common types: chronic lymphocytic leukemia (CLL), common in adults over 55 years old; acute lymphoblastic leukemia (ALL), prevalent in children (2-5 years); chronic myelogenous leukemia (CML), which affects mainly male subjects; acute myelogenous leukemia, which is more common in adults (INCA, 2013c).

CML was first described in 1845 by John Hughes Bennet (Joske, 2008). In the late 1960s, the researchers Nowell and Hungerford of the University of Pennsylvania identified an abnormal chromosome present in human leukemia cells (Geary, 2000). This chromosome, called Philadelphia (Ph), found in 95% of patients affected by CML, is the result of the reciprocal translocation between ABL1 gene in chromosome 9 and BCR gene in chromosome 22 [t(9;22)], leading to the formation of BCR-ABL oncoprotein

(Nowel *et al.,* 1961) (Druker et *al.,* 2001).

BCR-ABL hybrid protein, generated by the Ph chromosome, exhibits abnormal enzymatic activity of tyrosine kinase and is responsible for the pathogenesis of the disease (Daley et al., 1990). In normal conditions the tyrosine kinase regulates several fundamental activities, such as cell cycle, proliferation, differentiation, mobility and survival or cell death (Baselga, 2006).

In pathophysiology, BCR-ABL oncogene activates the protein tyrosine kinase. Upon binding with adenosine triphosphate (ATP), this protein transfers phosphate to tyrosine residues in specific proteins. These specific proteins, after phosphorylations, perform all the fundamental activities of a tyrosine kinase in an abnormal way, leading to the occurrence of CML (Druker *et al.,* 2001). Thus, blocking the binding between the protein and ATP is essential for disrupting all subsequent steps. The discovery of this mechanism was fundamental to the development of a target therapy for the treatment of patients with CML (Fardel *et al.,* 2000).

The first specific tyrosine kinase inhibitor tyrphostin was described in 1988 by Yaish *et al.* (Yaish *et aL,* 1988). Subsequently, some studies have identified the phenylaminopyrimidine (PAF) backbone as a potent nonspecific kinase inhibitor (Druker *et al.,* 2000; Choi *et al.,* 2010).

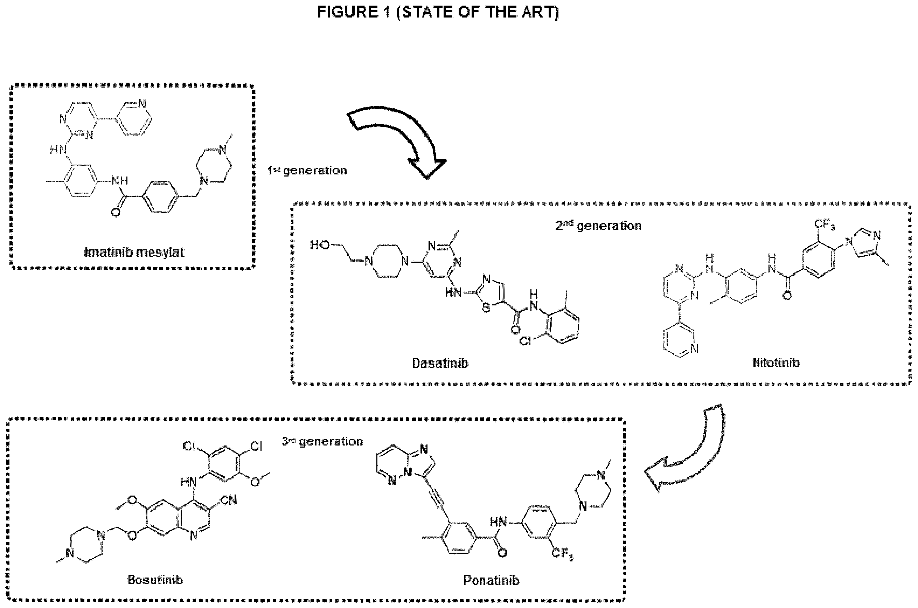

In 1993, Druker *et al.* synthesized and tested a series of molecules containing FAP-backbone (Capdeville *et al.,* 2002). The results identified that imatinib was capable of selectively eliminating CML cells (Goldman, 2000). With the advancement of clinical trials, this drug has come to be considered a "miracle". Statistics showed that almost 100% of the patients treated with imatinib reached the hematological remission of the disease (Hunter, 2007). Subsequently, new drugs were developed for the treatment of the disease and these were classified as inhibitors of second and third generation. The new inhibitors target more than one kinase and are called "multikinase" inhibitors. The second generation consists of dasatinib and nilotinib, and the third by bosutinib and ponatinib (Figure 1) (Quintas-Cardama *et al.,* 2010). Figure 1, incorporated herein as a reference, provides a summary of the first, second and third generation drugs used in CML chemotherapy.

In recent years, CML treatment has been revolutionized with the emergence of tyrosine kinase inhibitors, which has dramatically changed conventional therapy (Jabbour *et al.,* 2007). It has already been proven that these drugs induce high rates of cytogenetic and hematologic response, with a high overall survival rate. However, the success of this therapy has been hampered by the emergence of drug resistance and relapses.

Odaka Y. *et al.* (2014) teach that dihydroartemisinin (DHA) can inhibit rhabdomyosarcoma cells, a soft tissue (usually a muscle) tumor, present in the head, forehead, bladder, vagina, arms, legs and trunk of children. However, the molecular mechanism of this inhibition has not been elucidated. Furthermore, higher doses of DHA were shown to be cytotoxic to the cell when exposed to a period longer than 48 hours.

Document N°. WO2013/177420 relates to the use of DHA and its derivatives as inhibitors of BCR-ABL hybrid protein in leukemias, lymphomas, carcinomas and sarcomas. However, most of the evaluated compounds did not show significant inhibitory activity on resistant cells.

Holien T. *et al.* (2013) teach that the antitumoral activity of artesunate is verified in multiple myeloma cells and diffuse large B-cell lymphoma (DLBCL). These studies were carried out to treat cases of relapse and resistance to treatment. The results have indicated that doses of artesunate, commonly used in the treatment of malaria, are sufficient to inhibit myeloma and lymphoma cells. However, for the treatment of multiple myeloma, the effective dose within the bone marrow is more relevant. Moreover, there is no evidence of use in the treatment of resistance cases.

Zhou C. *et al.* (2012) teach that artesunate can induce apoptosis in human lung adenocarcinoma in ASTC-a-1 and A549 cells. The main objective of the study was to describe the mechanism of this therapeutic action. However, further research is needed to disclose the molecular targets of artesunate therapy, as well as its real therapeutic potential.

Jun Lee *et al.* (2013) teach that DHA, an active metabolite of artemisinin, can significantly inhibit the BCR-ABL gene in CML cells sensitive or resistant to imatinib, inducing cell death. It is worth mentioning that DHA is a compound of high commercial value when compared to sodium artesunate used in the present invention.

Thus, the development of new strategies is required to treat resistance cases in CML.

### SUMMARY OF THE INVENTION

The present invention relates to novel phenylaminopyrimidine(FAP)-derived compounds of general formulas I and II: 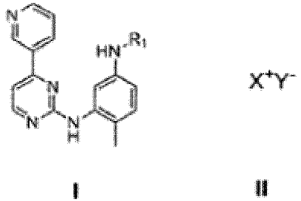 Where, in formula I, R₁ is: 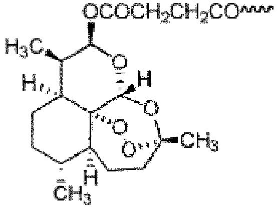 Where, in formula II, X⁺ is selected among one of the compounds below: 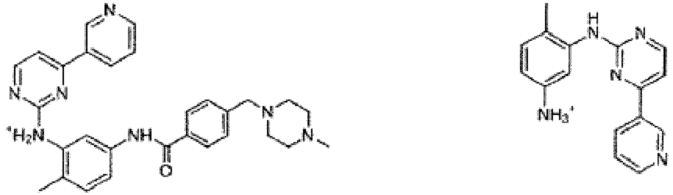 Where, in formula II, Y⁻ is

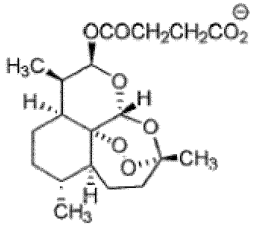

The compounds of formulas I and II of the present invention are powerful and nonspecific tyrosine kinase inhibitors being based on hybrid structures between artesunate with phenylaminopyrimidine (FAP) backbone and imatinib, which may be amides or salts thereof, respectively.

The process of obtaining the compounds of formulas I and II is also addressed in the present invention.

The invention further aims to use the novel phenylaminopyrimidine (FAP) derivatives of general formulas I and II in the treatment of patients with chronic myelogenous leukemia (CML) and other types of cancers whose treatment involves inhibition of the tyrosine kinase enzyme.

The present invention aims also to describe method for cancer treatment using the compounds of formulas I and II.

### BRIEF DESCRIPTION OF THE FIGURES

Figure 1 shows first, second and third generation drugs used in CML chemotherapy.
Figures 2A and 2B show a comparison of the action of the compounds of the invention in reducing the cell viability of K562 and K562 Lucena lineages.

### DETAILED DESCRIPTION OF THE INVENTION

The main object of the present invention is to provide novel substances derived from formulations I and II shown herein which, in addition of being active, do not induce the same resistance observed in the drugs already used in current therapy of the disease.

The compounds of the present invention are derived from phenylaminopyrimidine (FAP) of general formulas I and II: 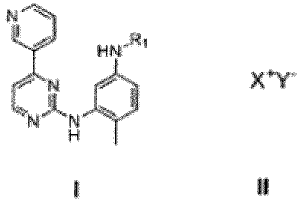 Where, in formula I, R₁ is: 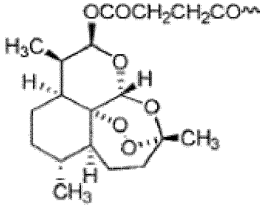 Where, in formula II, X⁺ is selected among one of the compounds below: 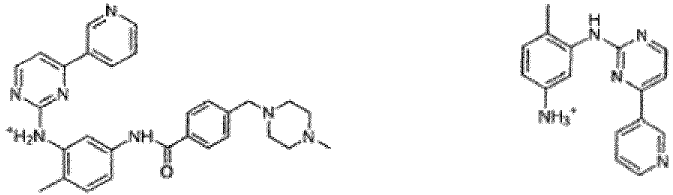 Where, in formula II, Y⁻

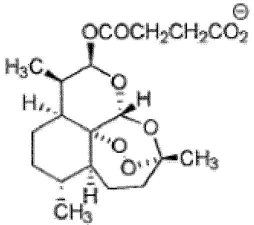

According to IUPAC, the compounds of formula I are called (3R,5aS,6R,8aS,9R,10R,12R,12aR)-3,6,9-trimethyldecahydro-3H-3, 12-epoxy[1,2]dioxopyne[4, 3-yl]isochromen-10-i1-4-((4-methyl-3-((4-(pyridin-3- yl)pyrimidin-2-yl)amino)phenyl)amino)-4-oxobutanoate. According to IUPAC, the compounds of formula II are called N-(2-methyl-5-(4-((4-methylpiperazine-1 -yl)methyl)benzamide)phenyl)-4-(pyridin-3 -yl)pyrimidin-2-aminium-4-oxo-4-(((3R,5aS,6R,8aS,9R, 10R, 12R, 12aR)-3,6,9-trimethyldecahydro-3H-3, 12- epoxy[1,2]dioxepin[4, 3-yl]isochromen-10-yl)oxy)butanoate and 4-methyl-3-((4-(pyridin-3-yl)pyrimidin-2-yl)amino)benzenaminium-4-oxo-4- (((3R,5aS,6R,8aS,9R,10R,12R,12aR)-3,6,9-trimethyldecahydro-3H-3,12- epoxy[1,2]dioxepin[4,3-yl]isochromen-10-yl)oxy)butanoate.

According to the present invention, the compounds 1-3, shown below, where obtained, said compounds being powerful nonspecific tyrosine kinase inhibitors, based on hybrid structures between artesunate with imatinib and with phenylaminopyrimidine (FAP) backbones, which may be salts or amides thereof.

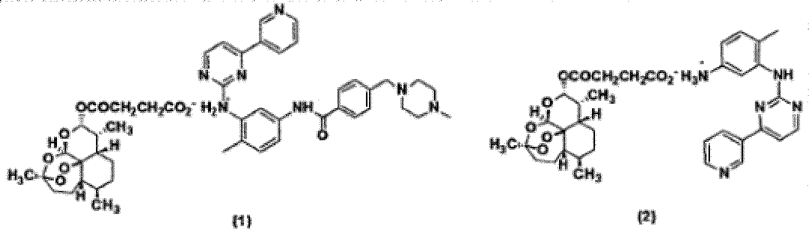

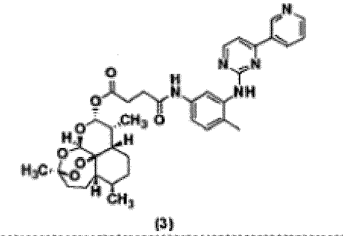

The reaction beginning with artesunate was proposed because the literature describes the growing importance of these derivatives for the treatment of several types of cancer, including CML. Furthermore, in the last few years, a wide variety of these biologically active derivatives have been synthesized and evaluated.

The method for obtaining compounds 1 to 3 of the present invention is a one-step process and the achieved yield is 84-88%, and is detailed in Example 1, below.

The invention will now be described on the basis of the examples, which are not to be construed as limiting the scope of protection.

### ORGANIC SYNTHESIS:

The synthesis strategy for the preparation of the compound of formula I (derivative 02) was initiated with the reaction between artesunic acid and PAF using EDC (1-hydroxybenzotriazole hydrate) and HOBT (1-ethyl-3 (3-dimethylaminopropyl) carbodiimide) as coupling agents. After 24 hours under stirring at room temperature, the desired product was formed with good reaction yields (Scheme 1).

The preparation of the novel hybrids of artesunate with imatinib and FAP, formula II (compound 03 and 04), was carried out by means of an acidic-base reaction between artesunic acid, imanitib bases and FAP, respectively (Scheme 1).

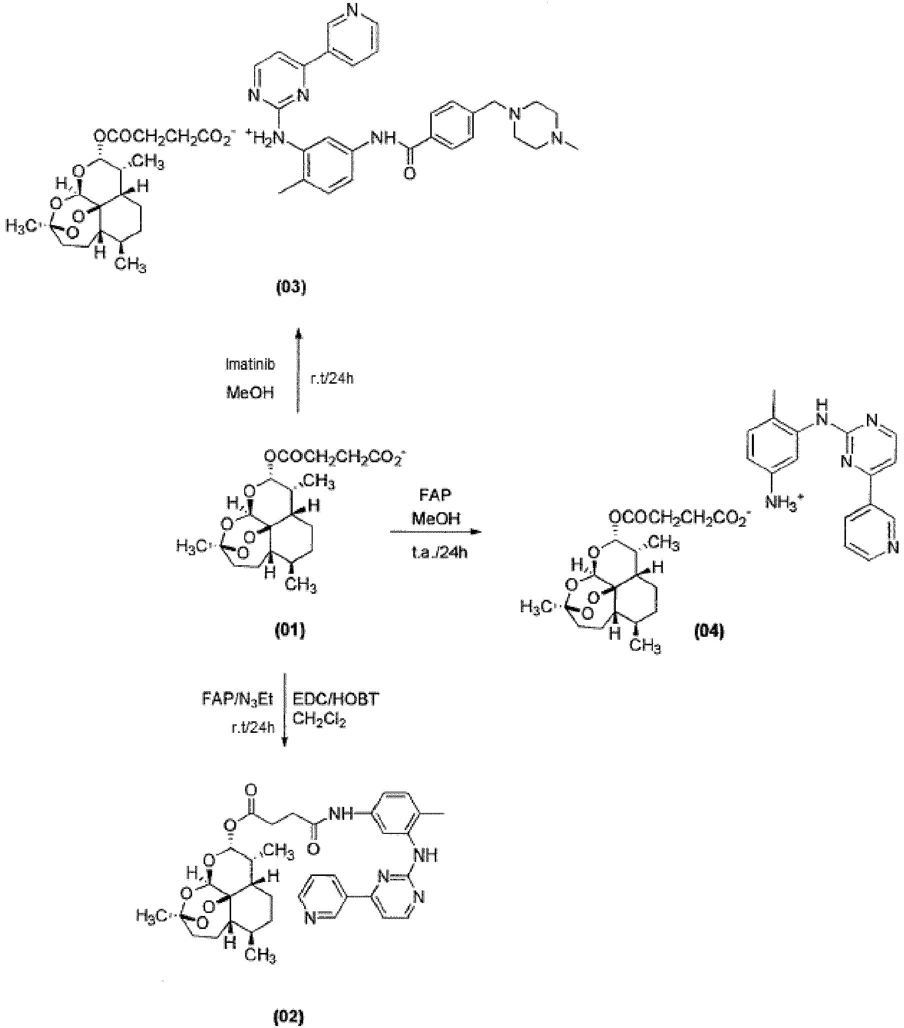

All the novel compounds were characterized by RMN ¹H and ¹³C, infrared and mass spectrometry.

Moreover, the compounds of the invention obtained according to Example 1 were evaluated for antineoplastic activity, cytotoxicity, and for the impact of the novel compounds on the cell cycle phases and induction of DNA fragmentation.

### ANTINEOPLASTIC EVALUATION:

The novel compounds of formula I-II, tyrosine kinase inhibitors (TKIs), have been tested on human cell lineages K562 and K562-Lucena 1, developed from K562, by continuous and increasing exposure to the chemotherapeutic vincristine. This lineage shows multidrug resistance (MDR) phenotype and exhibits the expression and activity of P-glycoprotein (Pgp) efflux pump. Both lineages K562 and K562-Lucena 1 express the BCR-ABL chimeric protein, constitutively activated, and derive from CML. Cell viability was evaluated through the MTT method, after 72 hours of incubation with TKIs. MTT is a colorimetric method which measures mitochondrial dehydrogenase activity, based on the ability of living cells to reduce the salt, 3-(4,5-dimethylazole-2-yl) -2,5-diphenyltetrazolium bromide in the product formazan.

### ANALYSIS OF SPONTANEOUS AND INDUCED APOPTOSIS FOSTER BY NOVEL COMPOUNDS IN LEUKEMIC CELLS:

The cytotoxicity assay, through annexin V labeling and flow cytometric analysis, was performed to determine the cytotoxic action of the novel compounds in K562 and K562-Lucena 1 lineages. Cells were incubated for 24 hours with or without addition of the novel compounds. The index of apoptosis induced by the novel compounds was obtained as follows: percentage of cells in induced apoptosis (treated with the drugs) minus the percentage of cells in spontaneous apoptosis (not treated with the drugs) (Vasconcelos *et aL,* 2007; Vasconcelos *et al.,* 2011).

### CELL CYCLE AND DNA FRAGMENTATION ANALYSIS:

The analysis of the impact of the new compounds on the phases of the cell cycle and on the induction of DNA fragmentation were carried out by DNA labeling with the fluorochrome propidium iodide. The assays were analyzed by flow cytometry and the results were analyzed in relation to the control (cells without treatment).

### EXAMPLE 1 - SYNTHESIS OF COMPOUNDS OF FORMULA I-(COMPOUND 02)

Amide **02** was obtained using 1 mol of artesunic acid, 1.8 mol of FAP, 0.5 mol of triethylamine, 1.5 mol of EDC coupling reagents and HOBT in 50 ml of dichloromethane. The medium was stirred at room temperature for 24 hours. At the end of the reaction, the organic phase was washed three times with a H₃PO₄ solution (5%), Na₂CO₃ saturated solution and water. The organic phase was dried with anhydrous sodium sulfate and evaporated, leading to the formation of the desired product.

### COMPOUND 02:

ESI-EM (3.00 Kv; 50 V), m/z (%): 643 (100).

### EXAMPLE 2 - SYNTHESIS OF COMPOUNDS OF FORMULA II-(COMPOUNDS 03-04)

The salts of the artesunic acid with imatinib **(03)** and FAP **(04)** were obtained using 1 mol of the respective free bases, which were solubilized in 10 ml of methanol. Artesunic acid (1 mol) in methanol (16 mL) was added to this solution. The reaction mixture was kept under stirring at room temperature for about 24 hours. After evaporation of the methanol and addition of pentane there was formation of a solid, which was dried under high vacuum.

### COMPOUND 03:

ESI-EM (3.00 Kv; 50 V), m/z (%): 877 (100).

### COMPOUND 04:

ESI-EM (3.00 Kv; 50 V), m/z (%): 661 (100).

### EXAMPLE 3 - ANTINEOPLASTIC EVALUATION

Cells were cultured in plates of 96 wells at the concentration of 1 x 10⁴ cells/ well in 200 µl of RPMI supplemented with 10% fetal bovine serum. 10 µl of each of the novel TKIs, or imatinib, previously diluted in RPMI medium, were added in different concentrations, and no molecule or imatinib was added in the control group. The plates were kept in a humid atmosphere containing 5% CO₂ at 37 °C for 72 hours. Four hours before completion of the established time (72 hours), 20 µl of MTT (Sigma) (final concentration of 10 µg/ml) were added. The plates were kept in the oven for the remaining four hours. 180 µl of the supernatant were removed from each well, and then 150 µl of dimethylsulfoxide (DMSO; Sigma) were added, homogenizing thoroughly for complete dissolution of the salt crystals formed by mitochondrial metabolism, thus resulting in a coloration. The obtained coloration has different apoptical densities, according to the mitochondrial metabolism treatment applications of the cells with the different concentrations of novel TKIs, or in the absence of these. The 96-well plate was read by the spectrophotometer (Spectramax Plus 384), using a wavelength of 570 nm. The results were analyzed through the absorbance of each well. The viability percentage was obtained through the following formula: [(Absorbance of cells treated with TKIs or imatinib/ Absorbance of untreated cells) x 100]. All the tests were performed in three different occasions. Three experiments, at least, independent, were carried out and triplicates were made for each concentration. By means of this method, we detected the cell inhibition percentage of each new molecule and we compared it with the percentage of viability reduction caused by imatinib.

The results showed that the novel compounds had similar or better activities when compared with imatinib. Moreover, the novel compounds did not exhibit cytotoxic activity. The results are described in Table 1.

**Table 1. Examples of biological evaluation of the novel compounds. Cell viability percentage after treating K562 lineage with different tyrosine kinase inhibitors. The results represent the average of three independent experiments.**

| | **3** | **4** | **Imatinib** |
|---|---|---|---|
| | **%** | **%** | **%** |
| **0.05µM** | 77.68 | 97.54 | 93.52 |
| **0.1µM** | 82.43 | 86.68 | 63.44 |
| **0.25µM** | 43.45 | 87.20 | 54.53 |
| **0.5µM** | 20.16 | 79.53 | 20.08 |
| **1.0µM** | 18.11 | 85.68 | 16.34 |
| **1.5µM** | 16.73 | 69.52 | 14.33 |
| **2.0µM** | 15.12 | 56.13 | 19.79 |
| **2.5µM.** | 17.89 | 53.28 | 19.43 |
| **3.0µM** | 16.69 | 48.95 | 14.75 |
| **4.0µM** | 16.51 | 48.17 | 13.53 |
| **5.0µM** | 15.76 | 47.69 | 15.01 |

Among the evaluated compounds, some had the same effect as that of imatinib. Compound **03** promoted a reduction in cell viability of about 50%, at a concentration of 0.25µM. The maximum reduction induced by these compounds was reached at the concentration of 1.0 µM, where approximately 20% of viable cells remained (Table 1). This percentage of viability remained constant despite the increase in the concentration of KIs molecules to the concentration of 5 µM (Table 1).

Compound **04** could induce a 50% reduction of viability, but to achieve this, a concentration of 2,5µM had to be used (Table 1). Although it is less effective than imatinib in reducing cell viability in K562 lineage, studies with this compound have continued to better evaluate its effect using other methodologies.

### EFFECTS ON THE NOVEL COMPOUNDS ON K562-LUCENA CELL LINEAGE

To investigate whether the novel compounds would be able to inhibit viability in a multidrug resistance (MDR) phenotype, with overexpression of the P-glycoprotein (Pgp), we initiated trials in the Lucena cell lineage. The compounds were tested for their cytotoxicity in this lineage. The action of these compounds was also compared with the action of imatinib.

The viability of the Lucena cell lineage was evaluated after 72h of incubation with the novel compounds, through the MTT assay.

Compound **03** was cytotoxic for at least 50% of the cells at a concentration of 0.25µM, while, 1.0µM of compound **04** were needed to attain a similar effect. However, the analyzed compounds were capable of reducing the viability of the Lucena cell lineage, in a dosage-dependent manner. These results suggest that such compounds have the propriety of overcoming the resistance barrier mediated by Pgp.

Upon incubation with imatinib, the viability reduction occurred in a concentration-dependent manner, until a plateau was reached, from a concentration of 1.5µM. The incubation of 0.25µM of imatinib led to approximately a 60% reduction in the viability of Lucena lineage. The novel TKIs compounds exhibit a cytotoxic effect similar to the one of imatinib.

### COMPARISON OF THE EFFECTS OF THE NOVEL COMPOUNDS IN THE REDUCTION OF CELL VIABILITY OF K562 AND K562-LUCENA LINEAGES

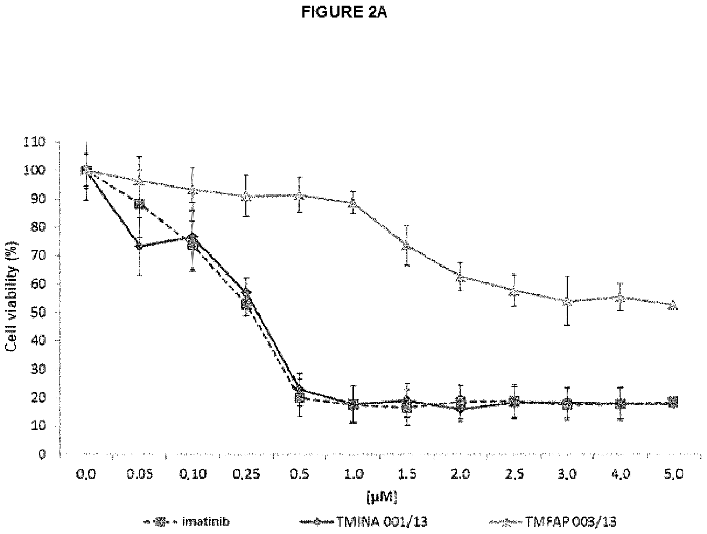

The action of the novel compounds was compared between the K562 lineage and the Lucena lineage. Compound **03** reduced the viability in the two lineages in a similar way. However, compound **04** was more effective in the Lucena lineage, where viability reduction was considerably higher than in the K562 lineage, after 72 hours of incubation, as shown in Figure **2A**.

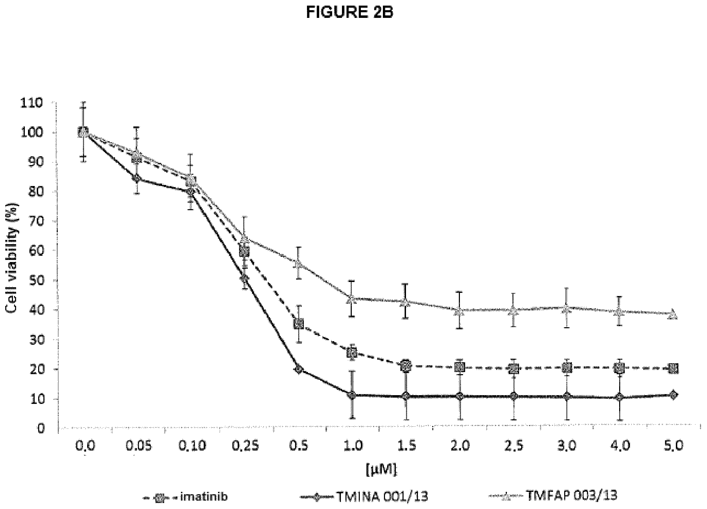

The action of these compounds was independent of the presence of Pgp, suggesting that they are not substrate for this carrier. On the other hand, the greater susceptibility of Lucena to compound **04** can suggest that this molecule enters the cell through an influx carrier, such as the OCT1 (Figure 2B). It was observed that this carrier is enhanced in the Lucena lineage when compared with the K562 lineage (Correa, 2012).

### CELL DEATH INDUCED BY IMATINIB AND BY THE NOVEL COMPOUNDS

To analyze the apoptosis induction potential by the new imatinib derivatives, annexin V/propidium iodide assay was used by flow cytometry.

To analyze death by apoptosis, the cells were incubated for 24 hours instead of 72 hours. Moreover, low concentrations of the compounds of the invention were chosen to proceed with the experiments (0.25µM, 0.5µM and 1.0µM).

Cells without addition of any drug were used for the evaluation of spontaneous death. Cells incubated with DMSO (used to solubilize the compounds derived from imatinib) were used to discard the effect of this substance on cell death induction. None of the novel compounds induced the lineages to death via necrosis (cells labeled only with propidium iodide). Compounds **03** and **04** induced cell death, probably by apoptosis (annexin-positive cells and annexin and propidium iodide-positive cells), in both lineages. Compound **04** induced death in a way similar to imatinib.

Upon incubation with compound **03**, the Lucena lineage showed a greater cell death rate than the K562 lineage. The Lucena lineage was more sensitive to compound **03** than the K562 lineage, differently from what has been observed in the MTT assay. In the present assay, the cells were incubated for 24 hours and cell death was analyzed. In the MTT assay, cell viability was verified after 72 hours of incubation. A plausible explanation for this divergence is that the response to compound **03** occurs immediately in the Lucena lineage and later in the K562 lineage.Regardless of the mechanism responsible for the cellular response to this compound, it is of undeniable importance because it induced death in cells of the K562-Lucena lineage, which have the MDR phenotype that is known to reduce the efficacy of imatinib, both in vitro and in clinical practice (Nestal de Moraes, 2012).

The treatment with compound **04** developed the opposite effect. Upon 24 hours of incubation with compound **04**, cell death rate did not reach the average of 20%, independently of the concentration and of the analyzed lineage. However, upon 72h of incubation, the Lucena lineage showed greater percentage of reduction of cell viability than the K562 lineage. It looks like compound **04** took more time to act and that it was capable to induce cell death only in the most resistant lineage. Cell death rate was higher with compound **04** than with imatinib in the Lucena lineage.

### INFLUENCE OF IMATINIB AND OF THE NOVEL COMPOUNDS ON CELL CYCLE AND DNA FRAGMENTATION

The induction of DNA fragmentation by the new compounds and by imatinib was assessed by flow cytometry. The analysis of cell cycle and DNA fragmentation was carried out upon 24 hours of incubation with the compounds.

In the K562 lineage, imatinib and compound **03** fostered a halt in the G0/G1 phase of the cell cycle, characterized by an increase of about 10% in the number of cells in that phase. In this lineage, compound **04** did not foster alterations in the cell cycle. Only imatinib induced DNA fragmentation in the K562 lineage.

Despite the low rate of cell death detected by the annexin/PI method, compound 03 induced DNA fragmentation in the Lucena lineage. Both assays were performed after 24 hours of incubation. Compound 03 probably induced DNA fragmentation by a path other than apoptosis. All the compounds induced halts at the G0/G1 phase. Cell increase at the G0/G1 phase fluctuated from 10 to 25%.

In combination, our data proved the effectiveness of molecules 03 and 04 in the induction of death in cells of CML which have different resistance mechanisms, such as BCR-ABL amplification and Pgp overexpression.

Considering the results presented herein, it is shown that the compounds of the present invention are capable of overcoming the resistance mediated by the drug-efflux carrier Pgp in chronic myelogenous leukemia cells. The novel compounds of the invention exhibit antineoplastic activity in resistant cells, which overexpress Pgp, more than the drugs used in the therapy of patients with CML, in addition to the fact that they do not exhibit cytotoxicity.

### REFERENCES (ACCORDING TO THE ORDER OF CITATION IN THE

### SPECIFICATION)

- Fauci, A. S.; Braunwald, E.; Kasper, D. L.; Hauser, S. L.; Longo, D. L.; Jameson, L.; Loscalzo, J. (2008) Harrison's Principles of Internal Medicine. McGraw-Hill.
- Rodriguez, M.; Cardona, A. F.; Enciso, L.; Ruiz, G.; Yepes, A.; Ospina, V.; Galvez, K.; Garcia, J.; Rosales, J.; Rosales, M.; Rosales, C.; Timana, J. L.; Casas, C. P.; Combariza, J. F.; Vargas, E.; Molano, A. (2006) Leucemia Mieloide crónica em crisis blástica: pespectiva terapêutica según la evidencia (parte II). Rev. Colomb. Cancerol., 10, 4, 267*.*
- Instituto Nacional de Cancer (INCA)^{b}. Coordenação Geral De Ações Estratégicas. Coordenação de Prevenção e Vigilância. Estimativa 2012: incidência de cancer no Brasil Instituto Nacional de Cancer Jose Alencar Gomes da Silva, Rio de Janeiro: Inca, 118, 2011. - Instituto Nacional do Cancer (INCA)^{c}. Acessed on May 28, 2013
- Joske, D. J. (2008) Chronic myeloid leukaemia: the evolution of gene-targeted therapy. Med. J. Aust., 1, 189, 277.
- Geary, C. G. (2000) The story of chronic myeloid leukaemia. British J. Haematol., 110, 1, 2.
- Nowell, P. C.; Hungerford, D. A. (1961) Chromosome studies in human leukemia. II. Chronic granulocytic leukemia. J. Natl. Cancer Inst., 27, 1013.
- Druker, B. J.; Sawyers, C. L.; Kantarjian, H.; Resta, D. J.; Reese, S. F.; Ford, J. M.; Capdeville, R.; Talpaz, M. (2001) Activity of a specific inhibitor of the BCR-ABL tyrosine kinase in the blast crisis of chronic myeloid leukemia and acute lymphoblastic leukemia with the Philadelphia chromosome. N. Engl. J. Med., 344, 14, 1038.
- Druker, B. J.; O' Dwyer, M. E. (2000) STI571: an inhibitor of the BCR-ABL tyrosine kinase for the treatment of chronic myelogenous leukaemia. Lancet. Oncol., 1, 207.
- Daley, G. Q.; Van Etten, R. A.; Baltimore, D. (1990) Induction of chronic myelogenous leukemia in mice by the P210bcr/abl gene of the Philadelphia chromosome. Science, 247, 4944, 824.
- Baselga, J. (2006) Targeting Tyrosine Kinases in Cancer: The Second Wave. Science, 312, 5777, 1175.
- Fardel, S.; Kantarjian, H. M.; Talpaz, M.; O'Brien, S. (2000) New treatment approaches for chronic myelogenous leukemia. Semin. Oncol., 27, 5, 578.
- Yaish, P.; Gazit, A.; Gilon, C.; Levitzki, A. (1988) Blocking of EGF-dependent cell proliferation by EGF receptor kinase inhibitors. Science, 242, 4800, 933.
- Capdeville, R.; Buchdunger, E.; Zimmermann, J.; Matter, A. (2002) Glivec (STI571, imatinib), a rationally developed, targeted anticancer drug. National Rev. Drug Disc., 1, 493.
- Choi, H. G.; Sim, T.; Gray, N.; Zhou, W.; Chang, J. W.; Zhang, J.; Weisberg, E. (2010) Fused heterocyclic compounds and their uses. WO2010144909.
- Goldman, J. M. (2000) Tyrosine-kinase inhibition in treatment of chronic myeloid leukaemia. Lancet, 355, 1031.
- Hunter, T. (2007) Treatment for chronic myelogenous leukemia: the long road to imatinib. J. Clin. Invest., 117, 8, 2036.
- Quintas-Cardama, A.; Kantarjian, H.; Cortes, J. (2010) Third-generation tyrosine kinase inhibitors and beyond. Semin. Hematol., 47, 4,371
- Jabbour, E.; Cortes, J. E.; Giles, F. J.; O'Brien, S.; Kantarjian, H. M. (2007) Current and emerging treatment options in chronic myeloid leukemia. Cancer, 109, 11, 2171.
- Odaka Y et al. (2014) [Dihydroartemisinin inhibits the mammalian target of rapamycin-mediated signaling pathways in tumor cells., Carcinogenesis, vol 35 No. 1, pp192-200, 2014, Odaka Y¹, Xu B, Luo Y, Shen T, Shang C, Wu Y, Zhou H, Huang S.]
- Holien T. et al. (2013) [Lymphoma and myeloma cells are highly sensitive to growth arrest and apoptosis induced by artesunate., European Journal of Haematology, 2013 Oct; 91(4):339-46. Holien T, Olsen OE, Misund K, Hella H, Waage A, Re3 TB, Sundan A.]
- Zhou C. et al., (2012) [Artesunate induces apoptosis via a Bak-mediated caspase-independent intrinsic pathway in human lung adenocarcinoma cells. Journal of Cellular Physiology, 2012, Dec; 227(12):3778-86. Zhou C, Pan W, Wang XP, Chen TS.]
- Jun Lee et al. [Biomedicine and pharmacotherapy (Biomedecine & pharmacotherapie), Vol. 67, n° 2, pages 157-163 (2013), "Dihydroartemisinin inhibits the Bcr/Abl oncogene at the mRNA level in chronic myeloid leukemia sensitive or resistant to imatinib", Jun Lee, Peiqiang Shen, Guobing Zhang, Xiuhua Wu, Xingguo Zhang]
- Vasconcelos, F. C.; Cavalcanti Jr, G. B.; Silva, K. L. (2007) Contrasting feactures of MDR phenotype in leukemias by using two fluorochromes: Implications for clinical practice. Leuk. Res., 31, 445.
- Vasconcelos, F. C.; Gattass, C. R.; Rumjanek, V. M.; Maia, R. C. (2007) Pomolic acid-induced apoptosis in cells from patients with chronic myeloid leukemia exhibiting different drug resistance profile. Invest New Drugs, 25, 6, 525.
- Vasconcelos, F.C.; Silva, K. L.; Souza, P. S.; Silva, L. F.; Moellmann-Coelho, A.; Klumb, C. E.; Maia, R. C. (2011) Variation of MDR proteins expression and activity levels according to clinical status and evolution of CML patients. Cytometry B Clin. Cytom., 80, 3,158.
- Correa, S.; Pizzatti, L.; Du Rocher, B. (2012) A comparative proteomic study identified LRPPRC and MCM7 as putative actors in imatinib mesylate cross-resistance in Lucena cell line. Proteome Sci. Mar., 30, 10, 23.
- Nestal de Moraes, G.; Souza, P. S.; Costas, F. C. (2012) The Interface between BCR-ABL-Dependent and -Independent Resistance Signaling Pathways in Chronic Myeloid Leukemia. Leuk. Res. Treatment., 2012, 671702.

## Claims

1. Compounds derived from phenylaminopyrimidine **characterized by** having the general formulas I and II: 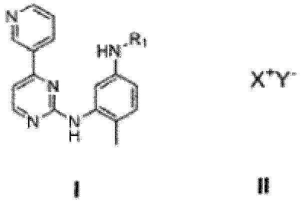 Where, in formula I, R₁ is 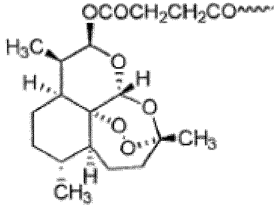 Where, in formula II, X⁺ is selected among one of the compounds below: 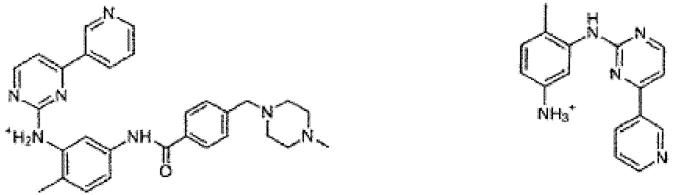 Where, in formula II, Y⁻ is:

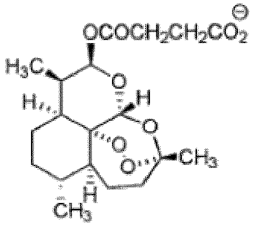

2. The compounds, according to clam 1, **characterized in that** the compounds of formula I are (3R,5aS,6R,8aS,9R,10R,12R,12aR)-3,6,9-trimethyldecahydro-3H-3-, 12-epoxy[1,2]dioxopyne[4, 3-yl]isochromen 10-yl-4-((4-methyl- 3-((4-(pyridin-3- yl)pyrimidin-2-yl)amino)phenyl)amino)-4-oxobutanoate.

3. The compounds, according to clam 1, **characterized in that** the compounds of formula II are N-(2-methyl-5-(4-((4-methylpiperazine-1-yl)methyl)benzamide)phenyl)-4-(pyridin-3-yl)pyrimidin-2-aminium-4-oxo-4-(((3R,5aS,6R,8aS,9R,10R,12R,12aR)-3,6,9-trimethyldecahydro-3H-3, 12-epoxy[1,2]dioxepin[4, 3-yl]isochromen-10-yl)oxy)butanoate and 4-methyl-3-((4-(pyridin-3-yl)pyrimidin-2-yl)amino)benzenaminium-4-oxo-4-(((3R,5aS,6R,8aS,9R,10R,12R,12aR)-3,6,9-trimethyldecahydro-3H-3,12-epoxy[1,2]dioxepin[4,3-yl]isochromen-10-yl)oxy)butanoate.

4. A process for obtaining compounds of formula I, as defined in claim 1, **characterized in that** it is carried out in a single step, in which a mixture of artesunic acid and phenylaminopyrimidine is left in the presence of a coupling agent, under stirring conditions, at room temperature, for 24 hours.

5. The process, according to claim 4, **characterized in that** the coupling agent is 1-hydroxybenzotriazole hydrate (EDC) and 1-ethyl-3(3-dimethylaminopropyl)carbodiimide) (HOBT).

6. The process for obtaining compounds of formula II, as defined in claim 1, **characterized in that** it is carried out in a single step through an acid-base reaction between the artesunic acid and the bases imatinib and phenylaminopyrimidine. The reaction is carried out in the presence of an alcohol, under stirring, at room temperature for 24 hours.

7. The process, according to claim 6, **characterized in that** the alcohol is methanol.

8. Use of compounds of formulas I and II, as defined in claim 1, **characterized in that** said use involves the inhibition of the tyrosine kinase enzyme.

9. Use, according to claim 8, **characterized in that** said use is for the treatment of chronic myelogenous leukemia.

10. Use of the compounds of formulas I and II, as defined in claim 1, **characterized in that** said use is in the production of a pharmaceutical composition for the treatment of cancers involving the inhibition of tyrosine kinase enzyme.

11. Use, according to claim 10, **characterized in that** said use is for the production of a pharmaceutical composition for the treatment of chronic myelogenous leukemia.

12. A method for treating cancer **characterized by** apoptosis induction of cancerous cells by administering a therapeutically effective amount of at least one of the compounds of formulas I and II, as defined in any one of claims 1-3.

13. The method, according to claim 12, **characterized in that** the treatment is against chronic myelogenous leukemia.

14. A method for treating cancer **characterized in that** the treatment involves the inhibition of tyrosine kinase enzyme, by administering a therapeutically effective amount of at least one of the compounds of formulas I and II, as defined in any one of claims 1-3.

15. The method, according to claim 14, **characterized in that** the treatment is against chronic myelogenous leukemia.
